# EUROPEAN PATENT SPECIFICATION

(11) **EP 2 245 976 B1**
(45) Date of publication and mention of the grant of the patent: **04.03.2015**
(21) Application number: 08869478.1
(22) Date of filing: 23.12.2008
(51) Int. Cl.: A47L 17/08, A47K 7/03, A47L 1/15, A47L 13/17

(54) **DUAL-SURFACE BASE ELEMENT FOR DISPOSABLE AND RECYCLABLE CLEANING ARTICLES**
BASISELEMENT MIT DOPPELTER OBERFLÄCHE FÜR EINWEG- UND RECYCLING-REINIGUNGSARTIKEL
ÉLÉMENT DE BASE À DOUBLE SURFACE POUR ARTICLES DE NETTOYAGE JETABLES ET RECYCLABLES

(30) Priority: 04.01.2008 ES 200800008 U
(43) Date of publication of application: 03.11.2010
(73) Proprietor: Juan Juan Massaguer, S.l., 08310 Argentona Barcelona (ES)
(72) Inventor: DE JUAN LLINAS, Marta, E-08310 Argentona (Barcelona) (ES)
(74) Representative: Isern-Jara, Nuria
(86) International application number: PCT/ES2008/000796
(87) International publication number: WO 2009/087246

(56) References cited:
- EP-A1- 0 872 206
- EP-A1- 1 808 116
- EP-A2- 0 086 355
- EP-A2- 1 259 210
- WO-A1-97/49326
- ES-U- 1 043 539
- US-A- 4 780 361
- US-A1- 2001 029 967
- US-A1- 2003 003 832
- US-A1- 2006 005 338

## Description

### OBJECT OF THE INVENTION

The present invention, as expressed in the title of this specification, relates to a base element for disposable cleaning articles aimed at providing a base body for embodying cleaning articles such as sponges, cloths or other articles that will provide two cleaning surfaces having different textures: a smooth surface and a rough surface, for different treatments, which is completely recyclable in plastic containers, an additional objective of the invention being that the corresponding cleaning article may be used for therapeutic, hygienic or cosmetic uses, such as cleaning the skin of hospital patients, applying medicines, cosmetics and other products through the skin.

### BACKGROUND OF THE INVENTION

Different cleaning articles for therapeutic, hygienic, hospital, domestic or other uses are known to exist, such as sponges, cloths and similar articles having two different layers connected by a connecting area, in such a manner that they provide a cleaning tool or article with two surfaces having different textures for different treatments or cleaning.

Additionally, it is also known the invention disclosed in document EP-A-1808116, which provides for a cleaning implement comprising modified open-cell foam, suitable for hard surface cleaning. In said document, a dual surface cleaning implement is described, which is constituted by two foam surfaces fixed together by a certain attachment. The invention described therein is specially suitable for industrial cleaning of hard surfaces, where soil or grease must be removed. This characteristic is guaranteed by the rigidity of the foams constituting such cleaning implement.

Known articles of this kind have drawbacks such that they are not completely recyclable in plastic containers. On the other hand, we are not aware of the existence in the state of the art of any base element for cleaning articles consisting of two nap layers with a different finish, as in the case of the base element of the present invention.

### DESCRIPTION OF THE INVENTION

In order to achieve the objectives and avoid the drawbacks indicated in the preceding sections, the invention consists of a base element for cleaning articles, such as sponges, cloths or other articles, for therapeutic, hygienic, hospital, domestic or other uses, said element having two different layers, namely a smooth layer and a rubbing layer, connected by a connecting area.

As a novelty, according to the invention, one of said layers, that which forms the front of the element, is an expanded hollow polyester nap layer with a thickness comprised between 1 and 300 mm, while the layer forming the rear of the element is a pressed and punched polyester nap layer having a thickness comprised between 0.5 and 15 mm, thereby forming a recyclable element in a plastic container.

In a preferred embodiment of the invention, the thickness of the layer forming the front of the base element is between 25 and 30 mm, while the thickness of the layer forming the rear is 1 mm.

Additionally, in the preferred embodiment of the invention, the connecting area alluded to is established using adhesive means compatible with therapeutic uses.

According to different embodiments, one or two of the aforementioned layers of the base element may include the impregnation of a product such as a soapy, medicinal, cosmetic, polishing, perfuming or other substance.

The aforementioned impregnation may be disposed on the layer or layers of the element, forming a framework of lines, a logo or any other ornamentation.

Based on the structure described, the base elements for cleaning articles of the present invention has advantages such that it embodies completely recyclable bodies in plastic containers, given the components it uses. Additionally, it provides cleaning articles compatible with therapeutic uses. Another advantage of the invention consists of the fact that it provides cleaning articles with two surfaces having different textures, namely a smooth layer and a rubbing layer, for applying different treatments.

For the purpose of helping to better understand this specification and forming an integral part thereof, a single figure is attached below wherein the object of the invention has been represented in an illustrative and non limiting manner.

### BRIEF DESCRIPTION OF THE FIGURE

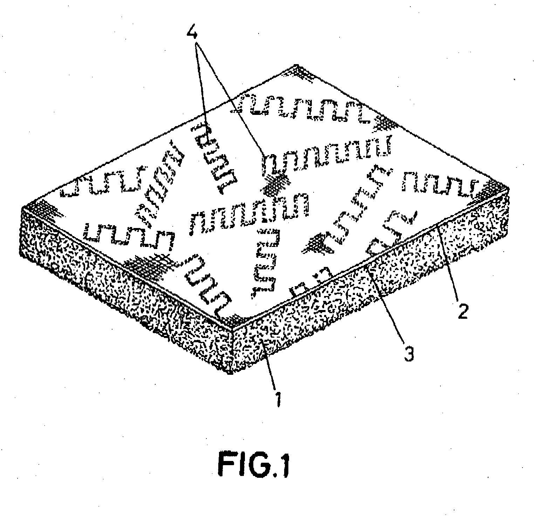

Figure 1 shows a perspective view of a base element for cleaning articles embodied according to the present invention.

### DESCRIPTION OF AN EXAMPLE OF EMBODIMENT OF THE INVENTION

A description of an example of the invention is provided below, making reference to the numbering adopted in the figure.

Therefore, the base element for cleaning articles of this example of the invention may be used to embody sponges, cloths and similar articles for therapeutic, hygienic, domestic cleaning or other uses; said element having two different layers with different thicknesses and textures that constitute a layer forming the front 1, a layer forming the rear 2 and a connecting area 3 that interconnects said layers 1 and 2, as shown in figure 1.

The layer forming the front 1 is an expanded hollow polyester nap layer with an envisaged thickness of between 1 and 300 mm, having a thickness of between 25 and 30 mm in this example.

The layer forming the rear 3 is a pressed and punched polyester nap layer, with an envisaged thickness of between 0.5 and 15 mm, having a thickness of 1 mm in this example.

On the other hand, the connecting area 3 is established using adhesive means compatible with the therapeutic uses.

The previously described constitution forms a recyclable element in a plastic container that favors the ecological aspect of the base element for cleaning articles of this example of the invention.

In figure 1, said base element adopts a rectangular configuration but, obviously, in other embodiments it may adopt other geometries.

Layer 1, layer 2 or both may include an impregnation 4 of a product such as a soapy, medicinal, cosmetic, polishing, perfuming or other substance which, in the present example, has been disposed in the form of a set of lines, as can be observed in figure 1, but in other examples this impregnated material 4 may be disposed forming a drawing, a logo or any other ornamentation.

## Claims

1. Dual-surface base element for disposable and recyclable cleaning articles, such as sponges, cloths or other articles for therapeutic, hygienic, hospital, domestic or other uses, said element having two layers with different textures, namely a smooth layer and a rough layer, connected by a connecting area (3), **characterized in that** one of said layers (1), forming the front of the element, is an expanded hollow polyester nap layer with a thickness comprised between 1 and 300 mm and having a rubbing texture; while the other layer (2), forming the rear of the element, is a pressed and punched polyester nap layer with a thickness comprised between 0.5 and 15 mm and having a smooth texture, thereby forming a recyclable element in a plastic container.

2. Dual-surface base element for disposable and recyclable cleaning articles, according to claim 1, **characterized in that** said thickness of the layer (1) forming the front is between 25 and 30 mm, while the thickness of the layer (2) forming the rear is 1 mm.

3. DUAL-SURFACE BASE ELEMENT FOR DISPOSABLE AND RECYCLABLE CLEANING ARTICLES, according to claim 1, **characterized in that** said connecting area (3) is established using adhesive means compatible with therapeutic and food handling-related uses.

4. Dual-surface base element for disposable and recyclable cleaning articles, according to claim 1, **characterized in that** one or two of said layers (1, 2) include an impregnation (4) of a product such as a soapy, medicinal, cosmetic, polishing, perfuming or other substance.

5. Dual-surface base element for disposable and recyclable cleaning articles, according to claim 4, **characterized in that** said impregnation (4) is disposed in the layer or layers (1, 2), forming a framework of lines, a logo or any other ornamentation.

## Patentansprüche

1. Basiselement mit doppelter Oberfläche für Einweg- und Recycling-Reinigungsartikel wie Schwämme, Tücher oder andere Artikel zu therapeutischem, hygienischem, Hospital-, Haushalts- oder anderem Gebrauch, wobei das besagte Element zwei Schichten mit verschiedener Beschaffenheit aufweist, nämlich eine glatte Schicht und eine raue Schicht, die durch eine Verbindungszone (3) verbunden sind, **dadurch gekennzeichnet, dass** eine der besagten Schichten (1), die die Vorderseite des Elementes bildet, eine Schicht aus geschäumtem hohlem Polyesterflor mit einer Dicke zwischen 1 und 300 mm ist, die eine reibende Struktur aufweist, während die andere Schicht (2), die die Rückseite des Elements bildet, eine Schicht aus gepresstem und perforiertem Polyesterflor mit einer Dicke zwischen 0,5 und 15 mm ist, die eine glatte Struktur aufweist, so dass es ein recyclingfähiges Element in einem Plastikbehälter bildet.

2. Basiselement mit doppelter Oberfläche für Einweg- und Recycling-Reinigungsartikel gemäss dem Anspruch 1, **dadurch gekennzeichnet, dass** die besagte Dicke der Schicht (1), die die Vorderseite bildet, zwischen 25 und 30 mm beträgt, während die Dicke der Schicht (2), die die Rückseite bildet, 1 mm beträgt.

3. Basiselement mit doppelter Oberfläche für Einweg- und Recycling-Reinigungsartikel gemäss dem Anspruch 1, **dadurch gekennzeichnet, dass** die besagte Verbindungszone (3) derart gestaltet ist, dass sie Haftmittel verwendet, welche mit therapeutischem Gebrauch und mit der Lebensmittelverarbeitung verbundenem Gebrauch verträglich sind.

4. Basiselement mit doppelter Oberfläche für Einweg- und Recycling-Reinigungsartikel gemäss dem Anspruch 1, **dadurch gekennzeichnet, dass** eine oder beide der besagten Schichten (1, 2) eine Imprägnierung (4) mit einem Produkt wie einer seifigen, medizinischen, kosmetischen, polierenden, parfümierenden oder anderen Substanz enthält.

5. Basiselement mit doppelter Oberfläche für Einweg- und Recycling-Reinigungsartikel gemäss dem Anspruch 4, **dadurch gekennzeichnet, dass** die besagte Imprägnierung (4) in der Schicht oder den Schichten (1, 2) enthalten ist und ein Gerüst von Linien, ein Logo oder ein beliebiges anderes Ornament bildet.

## Revendications

1. Elément de base à double surface pour des articles de nettoyage jetables et recyclables comme des éponges, tissus ou autres articles pour un usage thérapeutique, hygiénique, domestique, d'hôpital ou autre, ledit élément présentant deux couches avec des textures différentes, à savoir, une couche lisse et une couche rugueuse, connectées par une zone de connexion (3), **caractérisé par le fait que** l'une desdites couches (1), qui forme le devant de l'élément, est une couche d'un non-tissé expansé moussé de polyester avec une épaisseur comprise entre 1 et 300 mm, et qui présente une texture de frottement, tandis que l'autre couche (2), qui forme l'arrière de l'élément, est une couche d'un non-tissé pressé et perforé de polyester avec une épaisseur comprise entre 0,5 et 15 mm qui présente une texture lisse, de manière qu'elle forme un élément apte à être recyclé dans un récipient de plastique.

2. Elément de base à double surface pour des articles de nettoyage jetables et recyclables suivant la revendication 1, **caractérisé par le fait que** ladite épaisseur de la couche (1), qui forme le devant, est entre 25 et 30 mm, tandis que l'épaisseur de la couche (2), qui forme l'arrière, est 1 mm.

3. Elément de base à double surface pour des articles de nettoyage jetables et recyclables suivant la revendication 1, **caractérisé par le fait que** ladite zone de connexion (3) est réalisée de manière qu'elle utilise des moyens d'adhésion compatibles avec des usages thérapeutiques et relatifs au traitement des aliments.

4. Elément de base à double surface pour des articles de nettoyage jetables et recyclables suivant la revendication 1, **caractérisé par le fait que** l'une ou les deux des couches en question (1, 2) inclut une imprégnation (4) d'un produit tel qu'une substance savonneuse, médicinale, cosmétique, de polissage, de parfumage ou autre.

5. Elément de base à double surface pour des articles de nettoyage jetables et recyclables suivant la revendication 4, **caractérisé par le fait que** ladite imprégnation (4) est située dans la couche ou les couches (1, 2) et forme une structure de lignes, un logotype ou n'importe quelle autre ornementation.
